# EUROPÄISCHE PATENTSCHRIFT

(11) **EP 0 445 589 B1**
(45) Veröffentlichungstag und Bekanntmachung des Hinweises auf die Patenterteilung: **03.05.1995**
(21) Anmeldenummer: 91102495.8
(22) Anmeldetag: 21.02.1991
(51) Int. Cl.: C07C 209/48, C07C 211/09

(54) **Verfahren zur Herstellung von 1,4-Alkylendiaminen**
Process for the preparation of 1,4-alkylenediamines
Procédé pour la préparation d'alcylènediamines-1,4

(30) Priorität: 06.03.1990 DE 4006979
(43) Veröffentlichungstag der Anmeldung: 11.09.1991
(73) Patentinhaber: BASF Aktiengesellschaft, 67063 Ludwigshafen (DE)
(72) Erfinder: Koehler, Ulrich, Dr., W-6900 Heidelberg (DE); Siegel, Hardo, Dr., W-6720 Speyer (DE); Irgang, Matthias, Dr., W-6900 Heidelberg (DE)

(56) Entgegenhaltungen:
- BE-A- 682 595
- DE-A- 1 593 764
- DE-C- 902 616
- FR-A- 2 248 265
- US-A- 3 987 099

## Beschreibung

Diese vorliegende Erfindung betrifft ein neues Verfahren zur Herstellung von 1,4-Alkylendiaminen durch Umsetzung von Bernsteinsäuredinitrilen an Hydrierkatalysatoren, die Kobaltoxid und ein oder mehrere Oxide der Elemente Eisen, Nickel, Mangan, Chrom, Molybdän, Wolfram, Phosphor und ein oder mehrere Oxide der Alkali-, Erdalkali-, Seltenen Erdengruppe des PSE, von Scandium und/oder von Yttrium enthalten.

Aus der US-A 3 987 099 ist ein Verfahren zur Hydrierung von Nitrile, wie z.B. Ethylendinitril mit Wasserstoff bei Temperaturen von 60 bis 150°C und Drücken von 200 bis 350 Atm. (202 bis 354 Bar) an Hydrierkatalysatoren die Oxide von Co, Ni, Na, Fe, Ca und Mg enthalten, bekannt.

Aus der US-A-2 166 183 und der US-A-2 232 598 ist die diskontinuierliche Hydrierung von Bernsteinsäuredinitril an aktiviertem Kobaltpulver (im Autoklaven) mit Ausbeuten von 36 bzw. 70 % bekannt. Die JP 47/18809 und die JP 60/260544 geben für die diskontinuierliche Hydrierung von Bernsteinsäuredinitril an Kobalt-Katalysatoren in Gegenwart von Ca(OH)₂ und NH₃ bzw. NH₃ allein, Ausbeuten in Höhe von 68 % bzw. 73 % Butandiamin an. Ebenfalls Butandiaminausbeute von ca. 70 % werden für die diskontinuierliche Hydrierung von Bernsteinsäuredinitril an Ra-Co in Gegenwart von tertiären Aminen in der FR-A-2 248 265 beschrieben. Aus der JP 48/13305 und der JP 54/40524 sind Ausbeuten von 97 % bei der diskontinuierlichen Hydrierung von Bernsteinsäuredinitril an Ra-Co in Gegenwart von NH₃ und Mn bekannt. Dieses Verfahren arbeitet jedoch mit 130 g Katalysator auf 100 g Substrat in hoher Verdünnung und ist deshalb aufgrund seiner geringen Raumzeitausbeute zur Herstellung größerer Mengen Butandiamin ungeeignet.

Die DE-A-902 616 beschreibt eine halbkontinuierliche Fahrweise durch Zupumpen von Bernsteinsäuredinitril in THF zu einer Mischung aus Ra-Co, CaO, THF und NH₃ bei 200 bar H₂ (s. a. Houben Weyl, Bd. XI, I, S. 558-559). Die Ausbeuten betragen 89-93 %. Auch dieses Verfahren eignet sich nicht zur Herstellung größerer Mengen Butandiamin, da der Katalysator rasch inaktiv wird.

Aus der DE-A 954 416 ist die kontinuierliche Hydrierung von Bernsteinsäuredinitril an einem auf Kieselsäuresträngen aufgebrachtem, durch Zusatz von 0,25 % Phosphorsäure neutralisiertem, Kobaltkatalysator bekannt. Die Ausbeuten betragen ca. 92 %. Die DE-A-1 593 764 betrifft die kontinuierliche Hydrierung in Gegenwart von NH₃ an einem Katalysator, der aus 30 Gew.-% Kobalt auf Bimsstein besteht und mit einer 10 gew.-%igen wäßrigen Na₂CO₃-Lösung getränkt wurde. Die zuvorgenannten Katalysatoren zeichnen sich jedoch durch sehr rasche Inaktivierung aus. Die Standzeiten liegen unter 3 Tagen wie auch Vergleichsbeispiel 7 zeigt.

Es bestand deshalb die Aufgabe, ein Verfahren zu finden, das den zuvorgenannten Nachteilen abhilft.

Demgemäß wurde ein neues und verbessertes Verfahren zur Herstellung von 1,4-Alkylendiaminen der allgemeinen Formel I 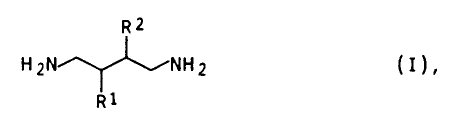
in der die Substituenten R¹ und R² unabhängig voneinander Wasserstoff, C₁- bis C₁₀-Alkyl, Phenyl oder Benzyl bedeuten, durch Umsetzung von Bernsteinsäuredinitrilen der allgemeinen Formel II 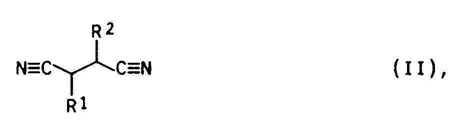
in der R¹ und R² die oben genannten Bedeutungen haben, mit Wasserstoff bei Temperaturen von 30 bis 250°C und Drücken von 50 bis 350 bar an Hydrierkatalysatoren gefunden, welches dadurch gekennzeichnet ist, daß die Hydrierkatalysatoren Kobaltoxid und ein oder mehrere Oxide der Elemente Eisen, Nickel, Mangan, Chrom, Molybdän, Wolfram, Phosphor und ein oder mehrere Oxide der Alkali-, Erdalkali-, Seltenen Erdengruppe des PSE, von Scandium und/oder von Yttrium enthalten.

Nach dem erfindungsgemäßen Verfahren arbeitet man im allgemeinen bei Temperaturen von 30 bis 250°C, vorzugsweise 50 bis 170°C, insbesondere 60 bis 150°C und Drücken von 50 bis 350 bar, vorzugsweise 150 bis 350 bar, wobei man einen Katalysator verwendet, der Kobaltoxid und ein oder mehrere Oxide, vorzugsweise ein bis drei der Elemente Eisen, Nickel, Mangan, Chrom, Molybdän, Wolfram, Phosphor und ein oder mehrere Oxide, vorzugsweise ein bis drei Oxide, besonders bevorzugt ein Oxid der Alkali-, der Erdalkali-, der Seltenen Erdengruppe, Scandium oder Yttrium enthält. Als Oxide der Elemente Eisen, Nickel, Mangan, Chrom, Molybdän, Wolfram, Phosphor eignen sich z.B. Fe₂O₃, NiO, Ni₂O₃, MnO₂, Mn₃O₄, MoO₃, CrO₃, Cr₂O₃, WO₃ und P₂O₅. Als Oxide der Alkali- oder Erdalkaligruppe kommen z.B. LiO₂, Na₂O, K₂O, MgO, CaO, SrO und BaO in Betracht. Als Oxide der Seltenen Erdengruppe eignen sich z.B. La₂O₃, Ce₂O₃, Gd₂O₃, Dy₂O₃, Sm₂O₃, Nd₂O₃ oder Er₂O₃. Unter katalytisch aktiver Masse versteht man diejenige Masse der genannten Metalloxide ohne Trägermaterialien. Die Katalysatoren können von 5 bis 100 Gew.-% aus katalytisch aktiver Masse bestehen.

Besonders geeignete Katalysatoren sind z. B. solche, deren katalytisch aktive Masse zu 20 bis 95 Gew.-%, vorzugsweise 40 bis 90 Gew.-% Kobaltoxid und 0,5 bis 60 Gew.-%, vorzugsweise 1 bis 40 Gew.-% aus einem oder mehreren Oxiden der Elemente Eisen, Nickel, Mangan, Chrom, Molybdän, Wolfram oder Phosphor, vorzugsweise einem oder mehreren Oxiden der Metalle Mangan, Nickel oder Eisen und 0,5 bis 20 Gew.-% aus einem oder mehreren Oxiden der Alkali- oder Erdalkali-, der Seltenen Erdengruppe, Scandium oder Yttrium bestehen.

Die Katalysatoren können als Vollkatalysatoren, z. B. als Granulat, oder auch aufgetragen auf inerte Träger, beispielsweise Siliciumdioxid, Aluminiumoxid, Zeolithe, Titandioxid, Magnesiumoxid, Bims, Zirkonoxid oder Kohlenstoff verwendet werden. Der Katalysator, der die metallischen Bestandteile in Form ihrer Oxide enthält, wird in der Regel vor einem Einsatz durch eine Wasserstoffbehandlung aktiviert, wobei die Oxide teilweise zu den Metallen reduziert werden.

Der zur Hydrierung verwendete Wasserstoff wird im allgemeinen im größeren stöchiometrischen Überschuß vom 1- bis 25-fachen, vorzugsweise vom 2- bis bis 10-fachen der stöchiometrisch notwendigen Menge verwendet. Er kann als Kreisgas in die Reaktion zurückgeführt werden. Der Wasserstoff kommt im allgemeinen technisch rein zum Einsatz. Beimengen von Inertgasen, z.B. Stickstoff, stören jedoch den Reaktionsablauf nicht.

Ein Zusatz von Ammoniak ist vorteilhaft, um die Bildung von sekundären Aminen, z.B. Pyrrolidinen weiter zurückzudrängen. Man arbeitet im allgemeinen mit Molverhältnissen von Ammoniak zu Bernsteinsäuredinitrilen von 1:1 bis 100:1, vorzugsweise von 2:1 bis 20:1.

Die Bernsteinsäuredinitrile der Formel II werden als Substanz oder als Lösung in einem geeigneten Lösungsmittel eingesetzt. Geeignete Lösungsmittel sind z.B. Ether, wie cyclische Ether, z.B. Tetrahydrofuran oder Dioxan, Alkohole, wie C₁-C₄-Alkohol, z.B. Methanol oder Ethanol oder andere für Hydrierungen an sich übliche Lösungsmittel, wie Formamiden, z.B. Dimethylformamid oder N-Methyl-pyrrolidon.

Die Hydrierung kann diskontinuierlich, vorzugsweise kontinuierlich am Festbett z.B. als Riesel- oder Sumpffahrweise durchgeführt werden. Bei diskontinuierlichem Betrieb verfährt man z.B. so, daß man ein Bernsteinsäuredinitril oder dessen Lösung zusammen mit dem Katalysator in einen Hochdruckautoklaven gibt, Ammoniak und Wasserstoff beide im Überschuß aufpreßt und das Reaktionsgemisch erhitzt. Nach Ende der Reaktion wird abgekühlt, der Katalysator abgetrennt und das Reaktionsgemisch fraktioniert destilliert.

Kontinuierlich verfährt man beispielsweise so, daß man in einen senkrecht stehenden Reaktor, der mit einem der genannten Katalysatoren beschichtet ist, von oben Bernsteinsäuredinitrile oder deren Lösung, Ammoniak und Wasserstoff in dem gewünschten Verhältnis zudosiert (Rieselfahrweise). Während der Reaktion werden die genannten Druck- und Temperaturbedingungen eingehalten. Am Reaktorausgang wird das Reaktionsgemisch gekühlt und in einem Abscheider in flüssige und gasförmige Bestandteile (Wasserstoff) zerlegt. Der flüssig Austrag wird von Ammoniak und Lösungsmittel befreit und destillativ gereinigt. Ammoniak, Wasserstoff und ein Teil des Rohaustrages können in den Reaktor zur Vervollständigung der Reaktion und Abführung der Reaktionswärme zurückgeführt werden.

Unter diesen Bedingungen gelingt es, 1,4-Alkylendiamine der Formel I in Ausbeuten > 97 % bei Katalysatorstandzeiten > 2 Monate zu erhalten.

1,4-Alkylendiamine sind wertvolle Zwischenprodukte. So findet beispielsweise 1,4-Butandiamin als Amin-Komponente in Nylon®-4,6 Verwendung.

### Beispiele

Die in den nachstehenden Beispielen genannten Prozente sind Gewichtsprozente.

### Beispiel 1

### Herstellung geeigneter Katalysatoren

### Katalysator A (Extrudate)

Eine wäßrige Lösung, die Kobaltnitrat, Eisennitrat und Mangannitrat enthält und eine 30 %ige wäßrige Natriumbicarbonatlösung werden über getrennte Pumpen so in einen Rührbehälter dosiert, daß ein konstanter pH-Wert von 6,5 eingehalten wird. Dabei wird die Temperatur im Fällungsbehälter auf 50°C gehalten.

Das Fällungsprodukt wird auf eine Filterpresse gepumpt und Na-frei gewaschen.

Die Filterpaste wird in einem Mischer mit Calciumhydroxid-Pulver (10 % CaO bezogen auf die Summe der Oxide) gemischt, getrocknet und bei 500°C calciniert. Das so entstandene Produkt wird in einem Kneter verdichtet und in eine verformbare Konsistenz übergeführt. Das Material wird extrudiert, getrocknet und nochmals bei 650°C calciniert. Die 4-mm-Extrudate haben folgende chemische Zusammensetzung (glühverlustfrei berechnet):
65,2 Gew.-% CoO
4,7 Gew.-% Mn₃O₄
10,0 Gew.-% CaO
20,1 Gew.-% Fe₂O₃

### Katalysator B (Extrudate)

Aus einer wäßrigen Lösung die Cobaltnitrat, Eisennitrat, Nickelnitrat und Mangannitrat enthält, wird in gleicher Weise wie bei Katalysator A beschrieben, mit Sodalösung das Katalysatorvorpordukt ausgefällt, filtriert und ausgewaschen.

Die Filterpaste wird in einem Mischer mit Calciumhydroxid-Pulver (10 % CaO bezogen auf die Summe der Oxide) gemischt, getrocknet und wiederum bei 500°C calciniert. Das so entstandene Produkt wird in einem Kneter verdichtet und in einer verformbaren Konsistenz übergeführt. Das Material wird extrudiert, getrocknet und nochmals bei 650°C calciniert. Die 4-mm-Extrudate haben folgende chemische Zusammensetzung (glühverlustfrei berechnet):
69,2 Gew.% CoO
4,9 Gew.% Mn₃O₄
5,5 Gew.% NiO
10,4 Gew.% Fe₂O₃
10,0 Gew.% CaO

### Beispiel 2

### Diskontinuierliche Hydrierung von Methylbernsteinsäuredinitril

50 g Methylbernsteinsäuredinitril II (R¹ = CH₃, R² = H), 50 g Tetrahydrofuran und 10 g des Katalysators A (in einem Maschendrahtkorb) werden in einem Autoklaven vorgelegt. Er wird mit Stickstoff gespült und 120 ml Ammoniak zugegeben. Man heizt auf 100°C auf und preßt 300 bar Wasserstoff auf, wobei stündlich Wasserstoff nachgepreßt wird. Nach Ende der Wasserstoffaufnahme, Abkühlen, Entspannen und Filtrieren und Einengen werden durch Destillation 52,0 g (95,8 %) Methylbutandiamin 1 (R¹ = CH₃, R² = H) erhalten.

### Beispiel 3

### Diskontinuierliche Hydrierung von Methylbernsteinsäuredinitril

Man verfährt wie in Beispiel 2 beschrieben, setzt jedoch einen Katalysator ein, der zu 65,3 % aus CoO, 5,2 % aus MnO, 10,3 % aus Fe₂O₃ und 19,2 % aus La₂O₃ besteht. Man erhält 53,9 g (99,4 %) Methylbutandiamin 1.

### Beispiel 4

### Diskontinuierliche Hydrierung von Bernsteinsäuredinitril

Man verfährt wie in Beispiel 3 beschrieben, setzt jedoch 50 g Bernsteinsäuredinitril II (R¹ = H, R² = H) ein. Es werden 54,2 g (98,5 %) Butandiamin 1 (R¹ = H, R² = H) erhalten.

### Beispiel 5

### Kontinuierliche Hydrierung von Bernsteinsäuredinitril

Ein Rieselreaktor mit der Länge von 3 m und einem Innendurchmesser von 16 mm wird mit dem Katalysator B (s. Beispiel 1) gefüllt. Am Kopfende des Reaktors wird eine 50 proz. Lösung von Bernsteinsäuredinitril II (R¹ = H, R² = H) in Tetrahydrofuran eingeleitet. Bei einem stündlichen Zulauf von 250 ml/h der Bernsteinsäuredinitril-Lösung, der unten angegebenen Menge Ammoniak und einem Wasserstoffdruck von 300 bar wird ein Flüssigkeitsumlauf von 9 l/h eingestellt. Es werden 3 Versuche bei unterschiedlichen Temperaturen durchgeführt. Die Austräge werden eingeengt und destilliert. Man erhält die in der nachstehenden Tabelle angegebenen Massenanteile:

| Temp. (°C) | Zulauf NH₃ (ml/h) | Butandiamin (%) | Pyrrolidin (%) | Rückstand (%) |
|---|---|---|---|---|
| 80 | 400 | 97,3 | < 0,5 | 1,2 |
| 100 | 400 | 98,5 | < 0,5 | 0,8 |
| 150 | 200 | 90,1 | 5,3 | 2,7 |

### Beispiel 6

### Kontinuierliche Hydrierung von Bernsteinsäuredinitril

Ein für die Sumpffahrweise geeigneter Reaktor mit einer Länge von 2 m und einem Durchmesser von 41 mm wird mit Katalyatorsträngen der Länge 7 mm und dem Durchmesser von 4 mm gefüllt. Der Katalysator enthält die folgenden aktiven Bestandteile: 70,3 % CoO, 10,5 % NiO, 5,2 % MnO, 14,0 % Na₂O. Am unteren Ende des R ktors wird eine 40 proz. Lösung von Bernsteinsäuredinitril II (R¹ = H, R² = H) in Methanol eingeleitet. Wasserstoff und Ammoniak werden ebenfalls von unten eingeleitet. Bei einem stündlichen Zulauf von 700 ml/h Dinitril-Methanol-Lösung, 500 ml/h Ammoniak und einem Wasserstoffdruck von 300 bar wird ein Umlauf von 40 l/h eingestellt. Man arbeitet den Austrag wie in Beispiel 5 geschildert auf. Bei 80°C werden folgende Massenanteile erhalten: Butandiamin 98,7 %, Pyrrolidin < 0,5 %, Rückstand 0,8 %.

### Beispiel 7 (Vergleichsversuche)

### Kontinuierliche Hydrierung von Bernsteinsäuredinitril - Vergleichsversuch

Der Rieselreaktor von Beispiel 5 wurde nacheinander mit dem Katalysator aus DE-A-954 416, Beispiel 3, DE-A-1 593 764, Beispiel 1, und dem Katalysator B, Beispiel 1 (diese Schrift) gefüllt, und jeweils mit 150 ml/h einer 50 proz. Lösung von Bernsteinsäuredintril (R¹ = H, R² = H) in THF, 240 ml/h Ammoniak beschickt bei einem Flüssigkeitsumlauf von 9 l/h, einer Temperatur von 100°C und einem Wasserstoffdruck von 300 bar. Nach Aufarbeitung wie in Beispiel 5 geschildert werden in Abhängigkeit von der Zeit folgende Butandiaminausbeuten erhalten:

| | Butandiamin (%) | | | |
|---|---|---|---|---|
| | nach 24 h | nach 48 h | nach 1 Woche | nach 4 Wochen |
| DE-A-954 416, Beispiel 1 | 78,2 | 65,3 | < 50 | - |
| DE-A-1 593 764, Beispiel 1 | 75,7 | < 50 | - | - |
| Katalysator B Beispiel 1 | 98,1 | 98,9 | 97,9 | 98,2 |

Die Standzeit des Katalysators B war größer als 2 Monate, während die beiden anderen Katalysatoren schon nach kurzer Zeit aufgrund von Verstopfungen zum Abstellen zwangen.

## Patentansprüche

1. Verfahren zur Herstellung von 1,4-Alkylendiaminen der allgemeinen Formel I 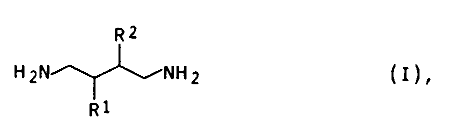 in der die Substituenten R¹ und R² unabhängig voneinander Wasserstoff, C₁- bis C₁₀-Alkyl, Phenyl oder Benzyl bedeuten, durch Umsetzung von Bernsteinsäuredinitrilen der allgemeinen Formel II 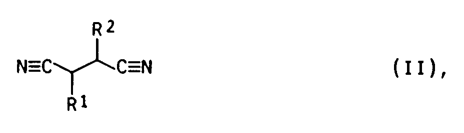 in der R¹ und R² die oben genannten Bedeutungen haben, mit Wasserstoff bei Temperaturen von 30 bis 250°C und Drücken von 50 bis 350 bar an Hydrierkatalysatoren, dadurch gekennzeichnet, daß die Hydrierkatalysatoren Kobaltoxid und ein oder mehrere Oxide der Elemente Eisen, Nickel, Mangan, Chrom, Molybdän, Wolfram, Phosphor und ein oder mehrere Oxide der Alkali-, Erdalkali-, Seltenen Erdengruppe des PSE, von Scandium und/oder von Yttrium enthalten.

2. Verfahren nach Anspruch 1, dadurch gekennzeichnet, daß die Hydrierkatalysatoren als katalytisch aktive Masse 20 bis 95 Gew.-% Kobaltoxid, 0,5 bis 60 Gew.-% Oxide der Metalle Mangan, Nickel, Eisen, Chrom, Molybdän, Wolfram oder Phosphor und 0,5 bis 20 Gew.-% Oxide der Alkali- oder Erdalkali-, der Seltenen Erdengruppe, Scandium oder Yttrium enthalten.

## Claims

1. A process for the preparation of a 1,4-alkylene-diamine of the general formula I 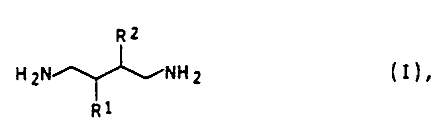 where R¹ and R², independently of one another, are each hydrogen, C₁-C₁₀-alkyl, phenyl or benzyl, by reacting a succinodinitrile of the general formula II 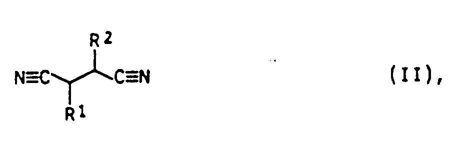 where R¹ and R² have the abovementioned meanings, with hydrogen at from 30 to 250°C and from 50 to 350 bar over a hydrogenation catalyst, wherein the hydrogenation catalyst contains cobalt oxide and one or more oxides of the elements iron, nickel, manganese, chromium, molybdenum, tungsten or phosphorus and one or more oxides of the alkali metal, alkaline earth metal or rare earth metal group of the Periodic Table of Elements, of scandium and/or of yttrium.

2. A process as claimed in claim 1, wherein the hydrogenation catalyst contains, as catalytically active material, from 20 to 95% by weight of cobalt oxide, from 0.5 to 60% by weight of oxides of the metals manganese, nickel, iron, chromium, molybdenum, tungsten or phosphorus and from 0.5 to 20% by weight of oxides of the alkali metal, alkaline earth metal or rare earth metal group, scandium or yttrium.

## Revendications

1. Procédé de préparation de 1,4-alkylènediamines de la formule générale I 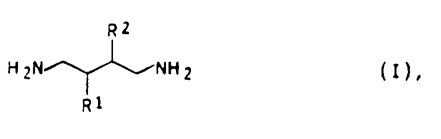 dans laquelle les substituants R¹ et R² représentent, chacun indépendamment l'un de l'autre, un atome d'hydrogène, un radical alkyle en C₁ à C₁₀, un radical phényle, ou un radical benzyle, par la réaction de dinitriles de l'acide succinique de la formule générale II 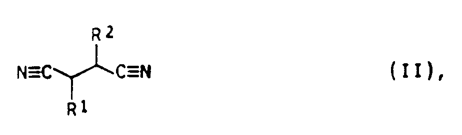 dans laquelle R¹ et R² possèdent les significations qui leur ont été attribuées ci-dessus, avec l'hydrogène, à des températures de 30 à 250°C et sous des pressions de 50 à 350 bars, sur des catalyseurs d'hydrogénation, caractérisé en ce que les catalyseurs d'hydrogénation contiennent de l'oxyde de cobalt et un ou plusieurs oxydes des éléments fer, nickel, manganèse, chrome, molybdène, tungstène, phosphore et un ou plusieurs oxydes des groupes des métaux alcalins, alcalino-terreux et des terres rares du SPE, du scandium et/ou de l'yttrium.

2. Procédé suivant la revendication 1, caractérisé en ce que les catalyseurs d'hydrogénation contiennent, à titre de masse catalytiquement active, 20 à 95% en poids d'oxyde de cobalt, 0,5 à 60% en poids d'oxydes des métaux manganèse, nickel, fer, chrome, molybdène, tungstène ou phosphore et 0,5 à 20% en poids d'oxydes des groupes de métaux alcalins, alcalino-terreux, des terres rares, du scandium ou de l'yttrium.
